# EUROPEAN PATENT SPECIFICATION

(11) **EP 0 563 414 B1**
(45) Date of publication and mention of the grant of the patent: **26.02.1997**
(21) Application number: 92105469.8
(22) Date of filing: 30.03.1992
(51) Int. Cl.: B01J 23/52, B01J 31/04, B01J 37/02, B01J 37/06, B01J 67/055

(54) **Process for producing a catalyst for the production of Alkenyl alkanoates**
Verfahren für die Herstellung eines Katalysators für die Bereitung von Alkenylalkanoaten
Procédé de préparation de catalyseur pour la production d'un alcanoate d'alkenyle

(43) Date of publication of application: 06.10.1993
(73) Proprietor: UNION CARBIDE CHEMICALS & PLASTICS TECHNOLOGY CORPORATION, Danbury, Connecticut 06817-0001 (US)
(72) Inventor: Bartley, William J., Charleston, West Virginia 25314 (US)
(74) Representative: Barz, Peter, Dr.

(56) References cited:
- EP-A- 0 403 950
- GB-A- 1 500 167
- US-A- 3 761 513
- US-A- 3 923 688
- US-A- 4 048 096

## Description

This invention relates to a process for producing catalysts (hereinafter referred to as "alkenyl alkanoate catalysts") for the production of alkenyl alkanoates from alkenes, alkanoic acids and an oxygen-containing gas.

Processes for producing alkenyl alkanoate catalysts are known.

By way of illustration, US-A-4,048,096 (Bissot) discloses a catalyst having a specific activity of at least about 83 grams of vinyl acetate per gram of precious metal per hour measured at 150°C. The Bissot vinyl acetate catalyst consists essentially of: (1) a catalyst support having a particle diameter of from about 3 to about 7 mm and a pore volume of from about 0.2 to about 1.5 ml./g., a 10% by weight water suspension of the catalyst support having a pH of from about 3.0 to about 9.0; (2) a palladium-gold alloy distributed in a surface layer of the catalyst support, the surface layer extending less than about 0.5 mm from the surface of the support, the palladium in the alloy being present in an amount of from about 1.5 to about 5.0 grams per liter of catalyst, and the gold being present in an amount of from about 0.5 to about 2.25 grams per liter of catalyst, and (3) from about 5 to about 60 grams per liter of catalyst of alkali metal acetate. Bissot discloses that the palladium is the active catalyst metal and the gold is a catalyst promoter.

Bissot also discloses a process for preparing the Bissot catalyst. Like the process of US-A-3,822,308 (Kronig et al.), the Bissot process involves precipitation of the metal salts on the catalyst support. The Bissot process comprises: (1) impregnating the catalyst support with aqueous solution of water-soluble palladium and gold compounds, (2) precipitating water-insoluble palladium and gold compounds on the catalyst support by contacting the impregnated catalyst support with a solution of compounds (preferably sodium metasilicate) capable of reacting with the water-soluble palladium and gold compounds to form water-insoluble palladium and gold compounds, (3) converting the water-insoluble palladium and gold compounds into palladium and gold metal on the support by treatment with a reducing agent, (4) washing the catalyst with water, (5) drying the catalyst (see Example 1 of Bissot), (6) impregnating the catalyst with alkali metal acetate promoter (e.g., a potassium promoter), and (7) drying the catalyst.

The improvement disclosed in Bissot involves distributing the palladium and gold as an alloy in a surface layer of the catalyst support, the surface layer extending less than about 0.5 millimeter from the surface of the support. The impregnating step is carried out with an aqueous solution of palladium and gold compounds and the total volume of the solution is from about 95 to about 100% of the absorptive capacity of the catalyst support. The precipitating step in Bissot is carried out by soaking the wet catalyst support with a solution of an alkali metal silicate, the amount of alkali silicate being such that, after the alkali metal silicate solution has been in contact with the catalyst support for about 12 to 24 hours, the pH of said solution is from about 6.5 to about 9.5.

Bissot does not report the sodium content of the catalysts of the Bissot Examples. Bissot Example 1 reports that the catalyst of that Example had an activity of 560 grams of vinyl acetate per liter of catalyst per hour. Two catalysts produced following the disclosure of Example 1 of Bissot were found to have sodium contents of 0.32 and 0.38 weight percent and activities of 551 and 535 grams of vinyl acetate per liter of catalyst per hour.

Despite the prior art processes, it is desirable to further improve the activity of alkenyl alkanoate catalysts.

In general, this invention provides a process for producing improved catalysts for the production of alkenyl alkanoates by the reaction of an alkene, an alkanoic acid and an oxygen-containing gas. The catalysts contain palladium, gold and a potassium promoter and are characterized by a reduced sodium content which results in increased catalyst activity.

This invention is based on the discovery that the activity of alkenyl alkanoate catalyst produced by the process of US-A-4,048,096 is increased if, after above-described step (7) of the process of that patent, the sodium content of the catalyst is reduced by washing the catalyst with water or with an aqueous solution of a potassium promoter.

More specifically, this invention provides a process for producing a catalyst that is useful in catalyzing the reaction of an alkene, an alkanoic acid and an oxygen-containing gas to produce an alkenyl alkanoate and that comprises support particles which are capable of exchanging cations and which are impregnated with palladium, gold and a potassium promoter selected from potassium alkanoates and any potassium compound that is converted to a potassium alkanoate during said reaction, said process comprising the steps of:
(a) impregnating the support particles with aqueous solutions of water-soluble palladium and gold compounds;
(b) precipitating water-insoluble palladium and gold compounds onto the support particles from such solutions using a precipitating agent;
(c) converting the precipitated water-insoluble palladium and gold compounds to palladium and gold on the support particles using a reducing agent;
(d) washing the impregnated support with water;
(e) drying the washed impregnated support;
(f) further impregnating the support particles with said potassium promoter;
(g) drying the support so impregnated to produce a dried catalyst containing sodium owing to the presence of sodium in one or more of the materials used in steps (a) to (f),
(h) washing the dried catalyst with water or with an aqueous solution containing said potassium promoter so as to reduce the amount of sodium in the catalyst to no more than 0.3 weight percent sodium based on the weight of the catalyst to increase the activity of the catalyst, and
(i) drying the catalyst.

In the practice of the process of this invention, it is preferred to use an aqueous solution containing the potassium promoter in step (h) to avoid lowering the concentration of the potassium promoter [with which the support was impregnated in step (f)] below the desired level. Such undesirable lowering of promoter concentration may occur if water as such is used in step (h). However, if water is used in step (h) and the promoter concentration is thereby undesirably decreased, then step (i) can be followed by step (j) which is a second potassium promoter impregnation and then by step (k) which is a third drying. In some instances, excess potassium promoter can be used in the initial potassium promoter impregnation [step (f)] to ensure that the product of step (i) has the desired level of potassium promoter even after water washing [step (h)]. The latter procedure also obviates the need for steps (j) and (k).

Without wishing to be bound by any particular theory, it is believed that the potassium promoter used in the catalyst preparation procedure of Bissot displaces at least a portion of the sodium which was bound to ion-exchange sites on the catalyst support. The source of the sodium is the starting materials (especially the precipitating agent) used in the Bissot (US-A-4,048,096) catalyst preparation procedure. Although displaced by the potassium promoter, such sodium remains in the catalyst produced by the Bissot process as an activity-suppressing impurity. In the process of this invention, the displaced sodium is readily removed from the catalyst by merely washing the catalyst with water or with an aqueous solution containing a potassium promoter [step (h)]. Prior to its displacement by the potassium promoter in step (f), the sodium cannot be effectively removed from the catalyst by the simple water washing [i.e., as in step (d)] because the sodium is too tightly bound to the support. However, step (d) is effective in removing unbound impurities, particularly chlorides and excess reagents from steps (a) through (c).

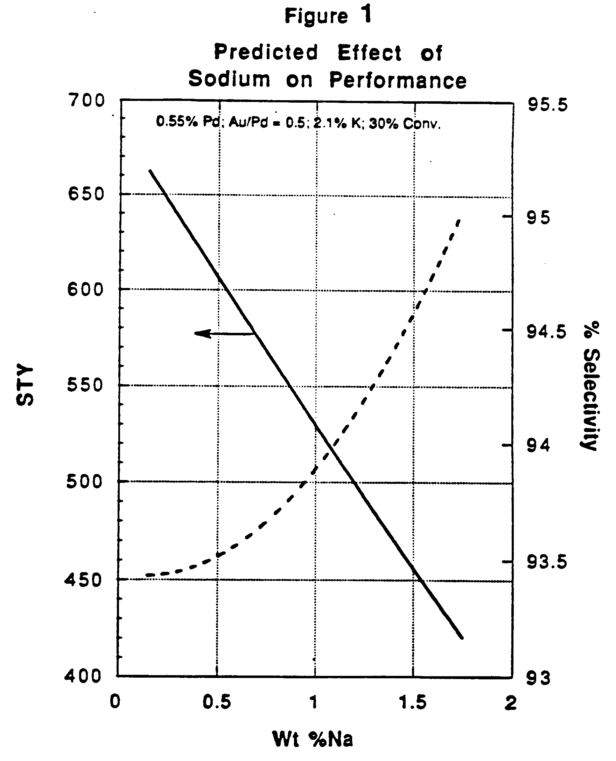

Figure 1 shows the predicted effect of sodium on the performance of vinyl acetate catalysts produced in accordance with this invention.

### DESCRIPTION OF THE PREFERRED EMBODIMENTS

The support particles used in the process of this invention are solid particulate materials that are capable of exchanging cations, that are capable of being impregnated with palladium, gold and a potassium promoter and that are inert under the conditions used to produce alkenyl alkanoates. Illustrative of such support particles are particulate silica, alumina, and silica-aluminas. Silica is the preferred support. The support preferrably has a surface area from 100 to 800 square meters per gram.

The aqueous solutions of water-soluble palladium and gold compounds used in the process of this invention include aqueous solutions of any suitable palladium or gold compound such as palladium (II) chloride, sodium tetrachloropalladium (II) (Na₂PdCl₄), palladium (II) nitrate, palladium (II) sulfate, gold (III) chloride or auric (III) acid (HAuCl₄). The volume of the solution preferably corresponds to from 95 to 100% (more preferably from 98 to 99%) of the pore volume of the support.

The precipitating agents used in the process of the present invention catalysts include sodium, lithium and potassium silicates and hydroxides. The precipitating agents are preferably employed in the form of aqueous solutions containing a 1.6 to 1.8 molar excess of the precipitating agents. The volume of such solutions used is preferably just sufficient to cover the support particles.

The reducing agents used in the process of this invention include hydrazine, ethylene, formaldehyde, hydrogen and sodium borohydride. The reducing agents are preferably employed in the form of aqueous solutions containing a 50:1 (or more preferably a 10:1) molar excess of the reducing agents. If hydrogen is used, it is usually necessary to heat the catalyst to 100 to 300°C to complete the reduction.

The potassium promoters used in the process Of this invention for producing alkenyl alkanoate catalysts are potassium alkanoates and any potassium compound that is converted to a potassium alkanoate during the alkenyl alkanoate-forming reaction (i.e., the reaction of ethylene, an alkanoic acid and an oxygen-containing gas in the presence of the catalyst to produce a alkenyl alkanoate). Suitable potassium compounds include potassium acetate, bicarbonate, nitrate and (when a stable support is used) hydroxide. The promoters are preferably applied in the form of aqueous solutions.

Washing steps (d) and (h) of the process of this invention can be conducted batchwise or continuously. Continuous washing is more efficient but may not be most suitable for large scale (e.g., plant scale) catalyst production. In continuous washing, the wash liquid is slowly and continuously passed through the catalyst over a period of time (e.g., from 8 to 24 hours). In batch washing, the catalyst is contacted with the wash liquid, the mixture is allowed to stand (e.g., for from 0.5 to 2.0 hours) and the liquid and catalyst are separated. In batch washing, several such washes (e.g., from 2 to 10, or preferably from 4 to 6 washes) are often required. Temperatures from 20°C to 80°C and volume ratios of wash liquid to catalyst of from 2:1 to 100:1 can be used in either batch or continuous washing.

The washing of the catalyst with water or an aqueous solution containing a potassium promoter in step (h) of the process of this invention is distinct from the potassium promoter impregnation steps of prior art processes for producing alkenyl alkanoate catalysts. Such prior art impregnation steps are conducted by the incipient wetness technique or the decantation technique. In the incipient wetness technique [see the Example 5 of GB-A-1,215,210 (National Distillers)], the catalyst is contacted with the minimum amount of aqueous potassium promoter solution required to fill the pores of the support and to impregnate the catalyst with the desired amount of potassium promoter. Then the water is evaporated. No sodium can be removed from the catalyst by that technique. In the decantation technique, the catalyst (preferably dry) is immersed in a larger volume of the aqueous potassium promoter solution than is used in the incipient wetness technique. After the pores are filled with the solution, the excess solution is decanted and the catalyst is dried. Only one immersion and decantation operation is conducted and the contact time is relatively short. Hence, only a minimal amount of sodium can be removed from the catalyst by the decantation technique. Example 9 of US-A-3,743,607 (Sennewald et al.) illustrates the decantation technique using a moist catalyst.

The drying of the catalyst in accordance with steps (e), (g), (i) or (k) of the process of this invention for producing alkenyl alkanoate catalysts can be conducted in any convenient manner. By way of illustration, drying can be conducted at 40°C to 120°C in a forced air oven for 15 to 30 hours.

In the following Examples, the following abbreviations are used:

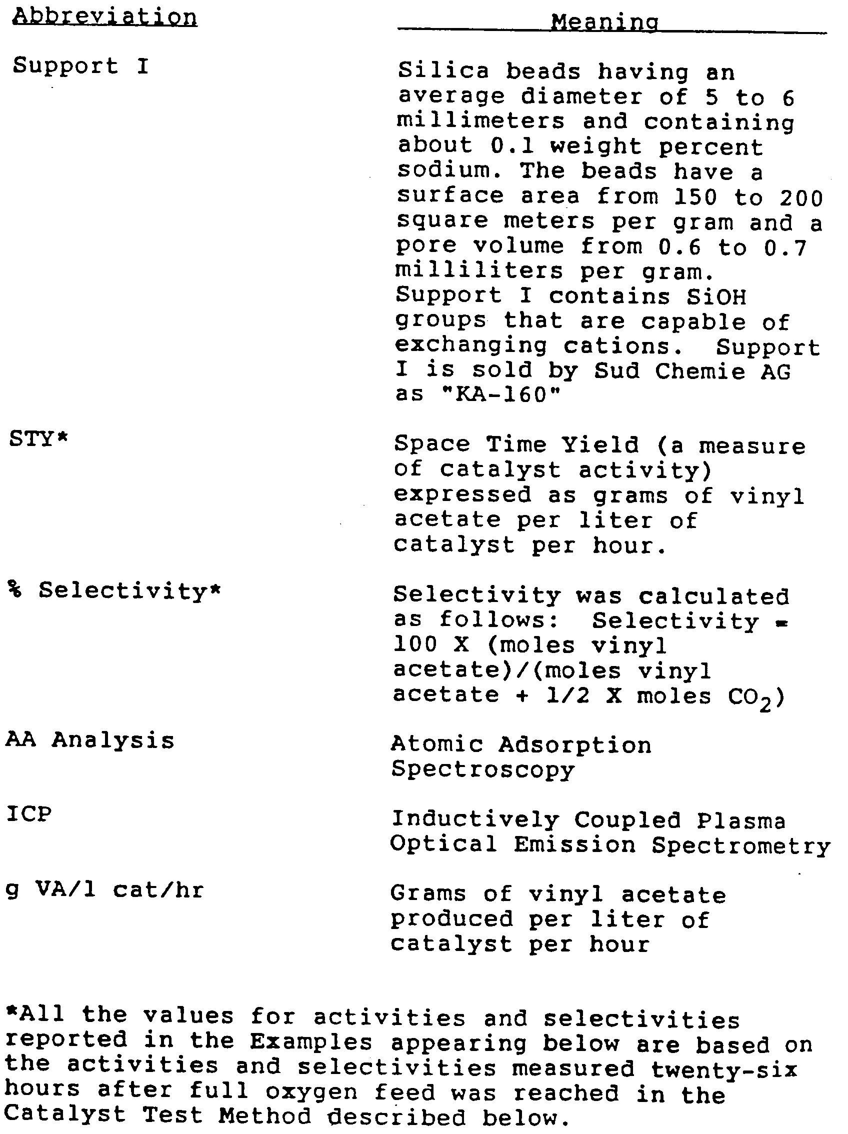

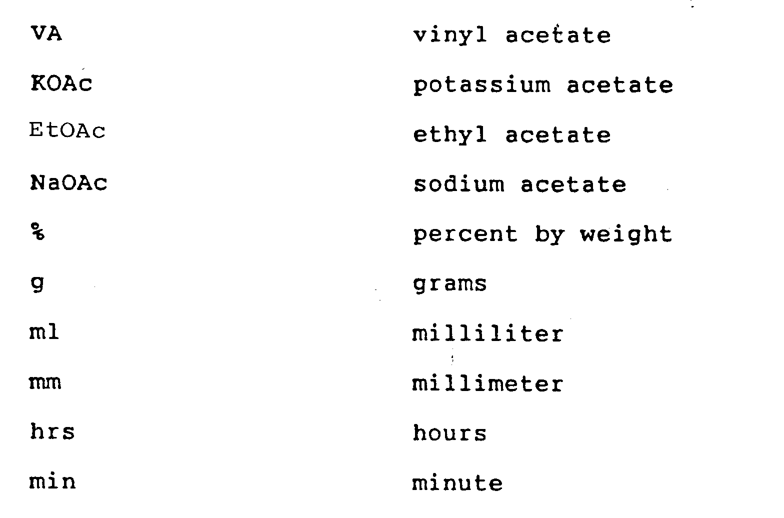

In the following Examples illustrating the present invention, the following procedures were used:

### Catalyst Preparation Procedure

A. Support I (15 g) was added to a solution of Na₂PdCl₄ (35.86% Pd, 0.258 g) and HAuCl₄ (48.95% Au, 0.094 g) dissolved in 9.0 ml of deionized water. The mixture so formed was gently agitated until all of the moisture was absorbed into the support and then was allowed to stand in a sealed flask for about one hour at room temperature so as to impregnate the support with the palladium and gold salts. The damp catalyst was covered with a solution of either sodium hydroxide (0.236 g in 28 ml water) or sodium metasilicate, Na₂SiO₃, (0.837 g in 28 ml water) as a precipitating agent. After mixing for a few seconds, the mixture was allowed to stand covered and undisturbed for 23 hours at room temperature to deposit water-insoluble palladium and gold compounds on the support. The palladium and gold were then reduced by the addition of 1.0 g of 85% hydrazine hydrate to the above mixture. The mixture was agitated for a few seconds and allowed to stand covered and undisturbed at room temperature for another 23 hours. The supernatant liquid was decanted from the catalyst and the catalyst was rinsed four times with water to remove the small amount of metal sludge present. The catalyst was washed thoroughly by either the Column Washing Procedure or the Batch Washing Procedure described below. The catalyst was dried on a stainless steel screen at 60°C in a forced air oven for 20 to 24 hours. The catalyst was analyzed for potassium using AA Analysis. Then the catalyst was impregnated with the desired amount of potassium acetate in water using the impregnation technique described above for the palladium and gold salts. Then the impregnated catalyst was dried at 60°C for 20 to 24 hours. The prior art process of US-A-4,048,096 (Bissot) [i.e., above described steps (a) to (g)] is complete at this point. The process of this invention has the additional steps (h) and (i) followed by, if desired, steps (j) and (k) as illustrated below.
B. The palladium, gold, sodium, and potassium contents in the finished catalysts were determined by ICP analyses. In most cases, sodium and potassium were also determined by AA Analyses for greater accuracy.
C. Unless otherwise noted, the foregoing procedure was used to prepare all the catalysts referred to in the following Examples. When different quantities of Support I were used, the quantities of the other starting materials used were varied correspondingly.
D. All the catalysts produced as described in the Examples appearing below were shell-impregnated (i.e., substantially all of the palladium and gold was present in a shell within 0.5mm of the surface of the beads of Support I.)

### Column Washing Procedure

Catalysts were washed or rewashed in a 1.24 inch o.d. x 24 inches glass chromatography column fitted with a Teflon™ stopcock. Typically 15 g of catalyst was added to the column which was then filled with water. The stopcock was adjusted to allow the liquid to flow from the column such that about one liter passed through the catalyst at room temperature over a period of about 24 hours. After this period, the excess liquid was drained from the column and the catalyst removed and dried as described above in the Catalyst Preparation Procedure.

### Catalyst Test Method

The catalyst (2.5 g samples of 5 to 6 mm catalyst spheres) was diluted with 10.5 ml of 0.5 mm glass beads and the mixture was uniformly distributed in both legs of a 316-stainless steel U-tube reactor. The reactor had an outside diameter of 0.95 cm (3/8 inch) and an overall height of about 15 cm (6 inches). An ethylene flow of 151 ml/min. was started through the reactor after which the catalyst was heated in an oven maintained at 150°C while allowing the system to pressurize to 0.79 MPa (115 psig). After maintaining at these conditions for 1.5 hours, acetic acid vapor was added to the ethylene and the mixture was passed over the catalyst for 45 minutes. Air was added to the feed gas at a slowly increasing rate over a 45-minute period until a total flow of 105 ml./min. was reached. The catalyst was allowed to stabilize for two hours before beginning data collection. The final gas composition was ethylene:acetic acid:oxygen:nitrogen = 52.9:10.7:7.7:28.7, the total gas hourly space velocity was about 3800 hr⁻¹, and the acetic acid liquid hourly space velocity was about 1 hr⁻¹. The product was analyzed by gas chromatography. The run-to-run reproducibility of the microreactors used in these experiments is about ±10 STY units.

### EXAMPLE I

### A. Comparative

An important step in alkenyl alkanoate catalyst preparation is the water washing step which is known to remove liberated chloride and residual starting materials. In the laboratory, catalysts are conveniently washed in a column over a 20 to 24-hour period, using about 60-80 ml of water per gram of catalyst. For practical and economic reasons, large-scale (e.g., pilot plant) preparations are washed batchwise over a much shorter time using significantly lower volumes of water per volume of catalyst washed. Catalysts made in large-scale equipment have been found to be less active than catalysts made in laboratory scale equipment. It was suspected that the activity differences were due to less efficient washing in the large-scale equipment.

Table I compares typical performance results of laboratory prepared catalysts with catalysts prepared in large-scale equipment following the process of US-A-4,048,096 (Bissot). Specifically, all the catalysts were made using the Catalyst Preparation Procedure with sodium metasilicate as the precipitating agent, except that the amounts of starting material was scaled up in the large-scale preparations and except for the differences in washing procedures. In the laboratory-scale preparations, the Column Washing Procedure was used and, in the large-scale preparations, the Batch Washing Procedure was used. The catalysts prepared using large-scale equipment include 80-liter pilot plant samples as well as samples from 260-liter equipment typical of that intended for full commercial production. The catalysts prepared using the large scale equipment typically contained about 5 to 10 weight percent more palladium than the catalysts prepared in the laboratory, yet the STY's of catalysts prepared in large-scale equipment are about 5 to 10 percent lower.

**TABLE I**

| Activities of Laboratory-Scale And Large-Scale Catalysts Preparations | | | | |
|---|---|---|---|---|
| Catalyst of Comparative Example | STY(a) | Spec. Act.(b) | %Pd(c) | %Au(c) |
| | | | | |

| Laboratory-Scale Preparations | | | | |
|---|---|---|---|---|
| I-1 | 571(3) | 197.0 | 0.516 | 0.198 |
| I-2 | 546(3) | 188.5 | 0.516 | 0.208 |
| I-3 | 543 | 191.6 | 0.505 | 0.198 |
| I-4 | 556(2) | 181.9 | 0.510 | 0.200 |
| I-5 | 547 | 194.4 | 0.500 | 0.190 |
| I-6 | 567 | 198.0 | 0.510 | 0.200 |
| Average | 555 | 191.9 | | |

| Large-Scale Preparations | | | | |
|---|---|---|---|---|
| I-7(d) | 521 | 165.4 | 0.558 | 0.231 |
| I-8(d) | 532(2) | 172.0 | 0.554 | 0.248 |
| I-9(d) | 534(2) | 172.7 | 0.553 | 0.238 |
| I-10(d) | 521(2) | 168.5 | 0.552 | 0.237 |
| I-11(e) | 495(6) | 163.0 | 0.543 | 0.214 |
| I-12(e) | 484 | 156.5 | 0.553 | 0.223 |
| I-13(e) | 485 | 156.9 | 0.554 | 0.222 |
| I-14(e) | 528 | 173.9 | 0.589 | 0.226 |
| I-15(e) | 525 | 172.8 | 0.568 | 0.217 |
| I-16(e) | 468 | 154.1 | 0.536 | 0.204 |
| I-17(e) | 489(2) | 155.5 | 0.562 | 0.213 |
| I-18(e) | 520 | 165.1 | 0.564 | 0.212 |
| I-19(e) | 479 | 149.6 | 0.568 | 0.203 |
| Average | 497 | 160.6 | | |

| | | | | |
|---|---|---|---|---|
| (a) STY = Space Time Yield, g VA/1 cat/hr, value in parentheses is the number of runs averaged; | | | | |
| (b) Specific Activity, g VA/g Pd/hr; | | | | |
| (c) Wt % Pd and Au as determined by ICP analyses; | | | | |
| (d) 80-liter pilot plant run; | | | | |
| (e) 260-liter plant-scale run. | | | | |

The data in Table I shows that the catalysts made on a large scale had lower activities than the catalysts made on a laboratory scale. To determine whether insufficient washing accounted for the lower activity of the catalysts made on a large scale, four of the catalysts made in large scale equipment (i.e., I-7, I-9, I-12 and I-17 of Table I) were rewashed (step (h) of the process of this invention) with water using the Column Washing Procedure and then dried [step (i) Embodiment B of the process of this invention]. A fifth catalyst was prepared on a laboratory scale using the Catalyst Preparation Procedure and the Batch Washing Procedure and a portion of this catalyst was rewashed with water (step (h) of the process of this invention) using the Column Washing Procedure and then dried [step (i) of the process of this invention]. Each of the five rewashed and redried catalysts was reimpregnated (step (j) of the process of this invention) with 5% potassium acetate to replace the potassium acetate assumed to have been removed during the rewashing and dried again [step (k) of the process of this invention ]. The results are shown in Table II and indicate that in every instance rewashing improved activity by 5 to 10 percent.

**TABLE II**

| Effect of Rewashing on Catalyst Activity | | | | |
|---|---|---|---|---|
| No. | Catalyst | Rewashed | STY | % Change |
| | | | | |

| Large Scale Preparations | | | | |
|---|---|---|---|---|
| 1* | I-7 | No | 521 | - |
| 2 | I-7 | Yes | 557 | +7 |
| 3* | I-9 | No | 534(2) | - |
| 4 | I-9 | Yes | 566 | +6 |
| 5* | I-12 | No | 484 | - |
| 6 | I-12 | Yes | 537 | +11 |
| 7* | I-17 | No | 489(2) | - |
| 8 | I-17 | Yes | 524 | +5 |

| Laboratory Preparation | | | | |
|---|---|---|---|---|
| 9* | | No | 508 | - |
| 10 | | Yes | 561 | +10 |

| | | | | |
|---|---|---|---|---|
| * Comparative Examples | | | | |

### EXAMPLE II

In the experiments described in Example I, it had been assumed that the rewashing (step (h) of the process of this invention) had also removed all the potassium acetate. Hence, in those experiments, potassium acetate had been reapplied (step (j) of the process of this invention) after the rewash in an amount equal to that used in the initial impregnation of the catalysts. The assumption made in connection with Example I was checked with another series of eight catalysts having varying potassium loadings. Analytical results obtained before and after rewashing are shown in Table III for these eight catalysts (Catalysts II-1 to II-8). These analytical results show that rewashing as described in Example I above did not, in fact, remove all the potassium acetate. The results shown in Table III indicate that about 0.9 weight percent potassium consistently remained in the catalyst after rewashing. It is believed that the potassium is bound to the support by an ion exchange mechanism.

**TABLE III**

| Rewashing vs KOAc Removal | | | |
|---|---|---|---|
| Catalyst | Wt% K | | |
| | Original | Rewashed(a) | Difference |
| II-1 | 2.80 | 3.67 | 0.86 |
| II-2 | 1.50 | 2.33 | 0.83 |
| II-3 | 1.40 | 2.27 | 0.87 |
| II-4 | 1.40 | 2.35 | 0.95 |
| II-5 | 2.70 | 3.51 | 0.81 |
| II-6 | 1.40 | 2.35 | 0.95 |
| II-7 | 2.90 | 3.64 | 0.73 |
| II-8 | 2.80 | 3.68 | 0.87 |
| | | | Average 0.86 |
| | | | %RSD(b) 8.30 |

| | | | |
|---|---|---|---|
| a) After reimpregnation with the same wt % KOAc as was present in the original catalyst. | | | |
| b) RSD is the Relative Standard Deviation. | | | |

### EXAMPLE III

In view of the findings shown in Table III, two of the rewashed catalysts shown in Table II were analyzed before reapplication of potassium acetate. Results, shown below, were generally consistent with those in Table III.

| Rewashed Catalyst | % K Retained |
|---|---|
| I-12 | 0.88 |
| I-17 | 1.19 |

As a consequence, the potassium loadings of all the rewashed catalysts reported in Table II were probably about 0.8 to 1.2% higher than anticipated. The higher than the desired potassium levels would be expected to decrease catalyst activity. This data suggests that activity improvements even greater than the observed 5-10% shown in Table II would be possible with proper (lower) final potassium loadings. This was confirmed when the amount of potassium added after rewashing was only the amount required to achieve the original loading as shown by Runs III-1 to III-7 below.

Comparative Run III-1: A 2.5 g portion of Catalyst II was evaluated for vinyl acetate production giving the result reported in Table IV. Portions of this catalyst were rewashed as described in Runs III-2 and III-3 below.

Run III-2: Three 50-g portions of catalyst from Run III-1 were each washed using the Column Washing Procedure and then analyzed for potassium which gave a potassium value of 0.92 wt. %. A 15-g sample of the catalyst was reimpregnated with 0.469 g of potassium acetate in 9.0 ml of water, then dried 18 hrs at 60°C. The analysis and test results for the catalyst are provided in Table IV.

Run III-3: The procedure of Run III-2 above was used, except that four samples of Catalyst II were used and each portion (200g) was washed with 7.6 ℓ (2 gallons) of water over a 48-hr period using the Column Washing Procedure. The potassium level was determined to be 0.86% after combining the washed material. A 15 g sample was impregnated with a solution of 0.556 g of potassium acetate in 9.0 ml of water then dried for 24 hrs at 60°C. The analysis and test results on the catalyst are shown in Table IV.

Comparative Run III-4: This catalyst was prepared using the Catalyst Preparation Procedure, except that the concentrations of the palladium and gold salts in the impregnation solution and the other starting materials were adjusted to give the loadings shown in Table IV. Evaluation was done using the Catalyst Test Method, except that only 0.75 grams of catalyst were used. The analysis and test results are shown in Table IV.

Run III-5: A 2.75 g portion of the catalyst from Run III-4 was washed using the Column Washing Procedure using 500 ml of water over 24 hours. After drying, the catalyst was impregnated with sufficient potassium acetate to give about 3% potassium in the finished catalyst. Evaluation was done using the Catalyst Test Method, except that only 0.75 grams of catalyst were used. The analysis and test results are shown in Table IV.

Comparative Run III-6: This catalyst was prepared as per Run III-4 above, except that the concentrations of the palladium and gold salts in the impregnation solution and the other starting materials were adjusted to give the loadings shown in Table IV. Evaluation was done using the Catalyst Test Method, except that only 0.75 grams of catalyst were used. The analysis and test results on the catalyst are provided in Table IV.

Run III-7: A sample of catalyst from Run III-6 was rewashed with water using the Column Washing Procedure. Evaluation was done using the Catalyst Test Method described above, except that only 0.75 grams of catalyst were used. The analysis and test results on the catalyst are provided in Table IV.

**TABLE IV**

| Effect of Rewashing at Constant Potassium Loading(a) | | | | | | | |
|---|---|---|---|---|---|---|---|
| Run | %Pd | %Au | %KOAc | % Na | Rewashed | STY | % Change |
| III-1* | 0.55 | 0.22 | 5.8 | 0.45 | No | 565 | --- |
| III-2 | 0.55 | 0.22 | 5.3 | 0.14 | Yes | 615 | +9 |
| III-3 | 0.5 | 0.22 | 5.8 | 0.12 | Yes | 642 | +14 |
| III-4* | 0.56 | 0.46 | 7.6 | 0.42 | No | 734(b) | --- |
| III-5 | 0.56 | 0.46 | 7.6 | 0.17 | Yes | 850(b) | +15 |
| III-6* | 1.02 | 0.46 | 7.2 | 0.48 | No | 967(b) | --- |
| III-7 | 1.02 | 0.47 | 7.7 | 0.17 | Yes | 1141(b) | +18 |

| | | | | | | | |
|---|---|---|---|---|---|---|---|
| (a) The Column Washing Procedure was used both in the original preparations and in rewashing. | | | | | | | |
| (b) This catalyst was tested at low conversion which accounts for the exceptionally high STY observed for this metal loading. | | | | | | | |
| * Comparative Examples. | | | | | | | |

On the basis of the results described above, studies were conducted to determine if other impurities might account for the described effects. Comparison of ICP analytical data from the original catalysts and their rewashed versions revealed significant differences in sodium content but no significant differences in other impurities were observed.

### EXAMPLE IV

To test the effect of sodium on catalyst activity, a series of experiments (runs) were conducted where sodium was varied and the potassium level was held constant. In another series of experiments (runs) the sodium to potassium ratio was varied while keeping the total moles of alkali constant. The results of both series of experiments are shown in Table V and the results confirm that increasing levels of sodium do, in fact, result in diminished activity.

In Run 2 of Table V, a rewashed catalyst which was impregnated with a level of sodium similar to that in the original unwashed version (Run 4 of Table V) showed an appreciably higher activity. This suggests that another detrimental impurity may also be removed by rewashing.

**TABLE V**

| Effect of Sodium on Catalyst Performance | | | | |
|---|---|---|---|---|
| Run No. | % K | % Na | STY | % Selectivity |
| | | | | |

| Constant K content & varied Na content (a) | | | | |
|---|---|---|---|---|
| 1 | 2.24 | 0.117 | 642 | 93.1 |
| 2 | 2.22 | 0.457 | 609 | 93.5 |
| 3 | 2.18 | 0.912 | 540 | 94.1 |
| 4 | 2.31 | 0.453 | 563 | 93.4 |

| Varied Na/K at constant moles alkali(b) | | | | |
|---|---|---|---|---|
| 1 | 2.21 | 0.435 | 616 | 93.6 |
| 2 | 1.77 | 0.705 | 584 | 94.0 |
| 3 | 0.77 | 1.300 | 501 | 94.2 |

| | | | | |
|---|---|---|---|---|
| (a) Column rewashed Catalyst II was reimpregnated with KOAc and NaOAc solutions. | | | | |
| (b) Prepared by appropriate addition of KOAc and NaOAc to subsamples of a master batch of Catalyst I. | | | | |

The catalysts obtainable by the process of this invention are useful in catalyzing the reaction of an alkene, an alkanoic acid and an oxygen-containing gas to produce an alkenyl alkanoate and comprise support particles which are capable of exchanging cations and which are impregnated with precipitated and reduced palladium and gold and a certain potassium promoter, any sodium in the catalyst being present in an amount of no more than 0.3 weight percent based on the total weight of the catalyst.

The catalysts obtainable by the process of this invention preferably have a palladium content of greater than 0.25 weight percent based on the total weight of the catalyst, more preferably greater than 0.5 weight percent based on the total weight of the catalyst and most preferably from 0.5 to 1.7 weight percent based on the total weight of the catalyst. It is preferred that the gold to palladium weight ratio of the catalyst is from 0.2 to 1.5 and, most preferably, from 0.4 to 1.2.

It is preferred that the catalysts obtainable by the process of this invention are shell-impregnated catalysts wherein a catalyst support has a particle diameter from about 3 to about 7 millimeters and a pore volume of 0.2 to 1.5 milliliters per gram. The palladium and gold are preferably distributed in the outermost 1.0 millimeter thick layer of the catalyst support particles. The catalysts preferably contain from about 1.4 to about 3.8 weight percent (more preferably from 2 to 3.6 weight percent) of potassium derived from the potassium promoter.

The catalysts obtainable by the process of this invention have reduced sodium contents. These catalysts contain no more than 0.3 weight percent sodium based on the weight of the catalyst. Preferably, the catalysts contain no more than 0.2 weight percent sodium and, most preferably, the catalysts contain no more than 0.1 weight percent sodium based on the weight of the catalyst. The amount of sodium in the catalysts obtainable by the process of this invention will depend upon such factors as the starting materials used, the number of washes, the total wash time, the volume of washing solutions and the concentration of the cation in the cation exchange solution.

The catalysts obtainable by the process of the present invention are used for producing alkenyl alkanoates. This comprises reacting an alkenyl, an alkanoic acid, and an oxygen-containing gas in the presence of a catalytic amount of catalyst as described above. The process is preferably conducted at a temperature from 100°C to 250°C (and most preferably at a temperature from 140°C to 200°C) and at a pressure from 0.1 MPa (15 psi) to 2.1 MPa (300 psi) (most preferably at a pressure from 0.6 MPa (90 pounds per square inch) to 1.0 MPa (150 pounds per square inch.)) The process is preferably conducted continuously in the vapor phase.

The above process for producing alkenyl alkanoates is characterized by the greater activity of the catalyst. Typically the activity of the catalysts is 5% to 25% greater (in terms of quantity of alkenyl alkanoate produced per unit of catalyst per unit time) as compared to otherwise identical catalysts containing from over 0.3 to about 1.0 weight percent sodium. Although catalyst selectivity (i.e., the tendency to produce alkenyl alkanoates rather than by-products such as carbon dioxide) declines somewhat with decreasing sodium content, that disadvantage is more than offset by increased catalyst activity, particularly in the range of sodium contents found in commercial alkenyl alkanoate catalysts (e.g., up to about 1.0 weight percent sodium).

Preferred alkanoic acid starting materials used in the above process for producing alkenyl alkanoates contain from two to four carbon atoms (e.g., acetic, propionic and butyric acid). Preferred alkene starting materials contain from two to four carbon atoms (e.g. ethylene, propylene and n-butene). Preferred products of the process are vinyl acetate, vinyl propionate, vinyl butyrate, and allyl acetate.

One aspect of the above process for producing alkenyl alkanoates involves the use of the models to predict the oxygen conversion to vinyl acetate, the activity of the catalyst, the selectivity of the catalyst to vinyl acetate, the production of ethyl acetate by-product and/or the production of heavy by-products.

The alkenyl alkanoates produced by the above process are known compounds having known utilities (e.g., vinyl acetate is useful in producing polyvinyl acetate).

## Claims

1. A process for producing a catalyst that is useful in catalyzing the reaction of an alkene, an alkanoic acid and an oxygen-containing gas to produce an alkenyl alkanoate and that comprises support particles which are capable of exchanging cations and which are impregnated with palladium, gold and a potassium promoter selected from potassium alkanoates and any potassium compound that is converted to a potassium alkanoate during said reaction, said process comprising the steps of:
(a) impregnating the support particles with aqueous solutions of water-soluble palladium and gold compounds;
(b) precipitating water-insoluble palladium and gold compounds onto the support particles from such solutions using a precipitating agent;
(c) converting the precipitated water-insoluble palladium and gold compounds to palladium and gold on the support particles using a reducing agent;
(d) washing the impregnated support with water;
(e) drying the washed impregnated support;
(f) further impregnating the support particles with said potassium promoter; and
(g) drying the support so impregnated to produce a dried catalyst containing sodium owing to the presence of sodium in one or more of the materials used in steps (a) to (f),
(h) washing the dried catalyst with water or with an aqueous solution containing said potassium promoter so as to reduce the amount of sodium in the catalyst to no more than 0.3 weight percent sodium based on the weight of the catalyst and thereby to increase the activity of the catalyst, and
(i) drying the catalyst.

2. A process as claimed in claim 1 wherein step (i) is followed by step (j) which is a second potassium promoter impregnation and by step (k) which is a third drying.

3. A process as claimed in claim 1 wherein an aqueous solution containing the potassium promoter is used in step (h).

4. A process as claimed in claim 1 wherein the catalyst produced by the process has a sodium content of no more than 0.2 weight percent based on the weight of the catalyst.

5. A process as claimed in claim 1 wherein the catalyst produced by the process has a sodium content of no more than 0.1 weight percent based on the weight of the catalyst.

## Patentansprüche

1. Verfahren zur Herstellung eines Katalysators, der für die Katalyse der Umsetzung eines Alkens, einer Alkansäure und eines sauerstoffhaltigen Gases unter Herstellung eines Alkenylalkanoats brauchbar ist und der Trägerteilchen umfaßt, die Kationen austauschen können und die mit Palladium, Gold und einem Kalium-Promotor, der aus Kaliumalkanoaten und einer beliebigen Kalium-Verbindung, die während der Umsetzung in ein Kaliumalkanoat umgewandelt wird, ausgewählt ist, imprägniert sind, wobei das Verfahren die folgenden Stufen umfaßt:
(a) Imprägnierung der Trägerteilchen mit wäßrigen Lösungen von wasserlöslichen Palladium- und Gold-Verbindungen;
(b) Abscheidung von wasserunlöslichen Palladium- und Gold-Verbindungen auf den Trägerteilchen aus derartigen Lösungen unter Verwendung eines Fällungsmittels;
(c) Umwandlung der abgeschiedenen wasserunlöslichen Palladium- und Gold-Verbindungen in Palladium und Gold auf den Trägerteilchen unter Verwendung eines Reduktionsmittels;
(d) Waschen des imprägnierten Trägers mit Wasser;
(e) Trocknen des gewaschenen, imprägnierten Trägers;
(f) weitere Imprägnierung der Trägerteilchen mit dem Kalium-Promotor; und
(g) Trocknen des so imprägnierten Trägers unter Herstellung eines getrockneten Katalysators, der auf Grund der Anwesenheit von Natrium in einem oder mehreren der in den Stufen (a) bis (f) verwendeten Materialien Natrium enthält,
(h) Waschen des getrockneten Katalysators mit Wasser oder mit einer wäßrigen Lösung, das bzw. die den Kalium-Promotor enthält, um die Menge an Natrium im Katalysator auf nicht mehr als 0,3 Gewichtsprozent Natrium, bezogen auf das Gewicht des Katalysators, zu vermindern und dadurch die Aktivität des Katalysators zu steigern, und
(i) Trocknen des Katalysators.

2. Verfahren nach Anspruch 1, in welchem auf Stufe (i) Stufe (j), bei der es sich um eine zweite Imprägnierung mit Kalium-Promotor handelt, und Stufe (k), bei der es sich um eine dritte Trocknung handelt, folgen.

3. Verfahren nach Anspruch 1, in welchem in Stufe (h) eine den Kalium-Promotor enthaltende wäßrige Lösung eingesetzt wird.

4. Verfahren nach Anspruch 1, in welchem der durch das Verfahren hergestellte Katalysator einen Natrium-Gehalt von nicht mehr als 0,2 Gewichtsprozent, bezogen auf das Gewicht des Katalysators, aufweist.

5. Verfahren nach Anspruch 1, in welchem der durch das Verfahren hergestellte Katalysator einen Natrium-Gehalt von nicht mehr als 0,1 Gewichtsprozent, bezogen auf das Gewicht des Katalysators, aufweist.

## Revendications

1. Procédé de préparation d'un catalyseur qui est utilisable pour catalyser la réaction d'un alcène, d'un acide alcanoïque et d'un gaz contenant de l'oxygène pour préparer un alcanoate d'alcényle et qui comprend des particules de support qui sont capables d'échanger des cations et qui sont imprégnées de palladium, d'or et d'un promoteur à base de potassium, choisi parmi des alcanoates de potassium et n'importe quel composé du potassium qui est transformé en alcanoate de potassium au cours de cette réaction, ce procédé comprenant les étapes consistant à :
(a) imprégner les particules de support de solutions aqueuses de composés du palladium et de l'or solubles dans l'eau ;
(b) précipiter les composés du palladium et de l'or insolubles dans l'eau sur les particules de support à partir de ces solutions en utilisant un agent de précipitation ;
(c) transformer les composés du palladium et de l'or insolubles dans l'eau précipités en palladium et en or sur les particules de support en utilisant un agent réducteur ;
(d) laver le support imprégné avec de l'eau ;
(e) sécher le support imprégné et lavé ;
(f) imprégner à nouveau les particules de support de ce promoteur à base de potassium ; et
(g) sécher le support ainsi imprégné pour préparer un catalyseur séché contenant du sodium en raison de la présence de sodium dans une ou plusieurs des matières utilisées dans les étapes (a) à (f),
(h) laver le catalyseur séché avec de l'eau ou avec une solution aqueuse contenant ce promoteur à base de potassium pour réduire la quantité de sodium dans le catalyseur à 0,3 % en poids de sodium au maximum par rapport au poids du catalyseur, et augmenter ainsi l'activité du catalyseur, et
(i) sécher le catalyseur.

2. Procédé selon la revendication 1, dans lequel l'étape (i) est suivie de l'étape (j) qui est une seconde imprégnation par un promoteur à base de potassium et par l'étape (k) qui est un troisième séchage.

3. Procédé selon la revendication 1, dans lequel une solution aqueuse contenant le promoteur à base de potassium est utilisée dans l'étape (h).

4. Procédé selon la revendication 1, dans lequel le catalyseur préparé par le procédé a une teneur en sodium ne dépassant pas 0,2 % en poids par rapport au poids du catalyseur.

5. Procédé selon la revendication 1, dans lequel le catalyseur préparé par le procédé a une teneur en sodium ne dépassant pas 0,1 % en poids par rapport au poids du catalyseur.
